# FASCICULE DE BREVET EUROPEEN

(11) **EP 2 404 568 B1**
(45) Date de publication et mention de la délivrance du brevet: **17.04.2013**
(21) Numéro de dépôt: 11354037.1
(22) Date de dépôt: 07.07.2011
(51) Int. Cl.: A61C 5/00

(54) **Dispositif de contention dentaire photopolymérisable**
Fotopolymerisierbare Zahnhaltevorrichtung
Photopolymerisable dental setting device

(30) Priorité: 07.07.2010 FR 1002861
(43) Date de publication de la demande: 11.01.2012
(73) Titulaire: Collombin, André, 38500 Voiron (FR)
(72) Inventeur: Collombin, André, 38500 Voiron (FR)
(74) Mandataire: Hecké, Gérard

(56) Documents cités:
- EP-A1- 0 666 061
- WO-A1-01/50979
- DE-A1-102005 023 282
- FR-A1- 2 815 352

## Description

### Domaine technique de l'invention

L'invention est relative à un dispositif de contention dentaire photo polymérisable, comprenant un élément allongé en matériau composite à base de fibres et de particules préimprégnées avec une résine, ledit élément étant conformé en attelle de contention destinée à solidariser plusieurs dents du patient.

### État de la technique

Le document US 2 755 552 divulgue des éléments de contention constitués de fibres pour constituer des attelles de contention invisibles, et sans couleur.

Le document FR 2 588 181 décrit des éléments de contention constitués de fibres haute résistance incluses dans des résines sous forme de matériaux composites, les fibres étant de toutes natures, verre, céramique, bore, carbure de bore, carbure de silicium, fibre synthétiques sous forme de faisceaux de fibres, parallèles, éventuellement guipées, tissus, tricots, tresses.

La résine se trouve sous forme partiellement ou totalement polymérisée, et il peut s'agir de résine époxy, polyester, ou n'importe quelle autre résine pourvu qu'elle soit compatible avec les fibres utilisées.

Les documents US 4717341 et US 4894012 décrivent des éléments semblables pouvant être utilisés en tant qu'éléments actifs ou passifs en orthodontie et en prothèse dentaire. Ces éléments sous forme d'éléments allongés, sont notamment proposés pour solidariser des dents entre elles. Ces éléments peuvent être proposés en l'état d'avant polymérisation et on parle de préimprégnés ou prepegg.

Les éléments sont collés sur les surfaces dentaires au moyen de colles composites photopolymérisables. On applique l'élément sur la surface dentaire par différents moyens connus. On utilise généralement une spatule pour maintenir l'élément sur la surface dentaire, et on insole l'élément et la colle pour durcir l'ensemble. Une autre technique consiste à maintenir sur la dent l'élément au moyen d'une bandelette transparente aux rayons lumineux, laquelle bandelette est appliquée par des moyens mécaniques, fils de suture, pinces, ou encore au moyen d'une gouttière transparente préformée préparée au laboratoire. Dans ce cas, la bandelette ou la gouttière n'adhèrent jamais aux surfaces dentaires.

La société 3M commercialise pour les soins cutanés sous l'appellation Steri Strip des bandelettes de sparadrap microporeux renforcé de fibres de polyester avec un adhésif hypoallergénique, présentées en pochette stérile. Ces bandelettes sont destinées à fermer une plaie sans utiliser de suture. Ces bandelettes ne sont pas destinées à servir d'attelles de contention dentaire, et ne renferment aucun matériau photopolymérisable.

La société COMPEED commercialise un pansement protecteur cutané, ou porte médicament adhésif, occlusif, destiné au traitement et à la prévention des plaies cutanées.

Le document WO-A-0150979 décrit un ensemble de contention dentaire comprenant un élément de contention allongé en matériau composite à base de fibres et de particules préimprégnées avec une résine photopolymérisable, et destiné à former après polymérisation, une attelle de renforcement de plusieurs dents entre elles. L'ensemble comporte un applicateur souple et déformable, et réalisé en un matériau conducteur de la lumière, tel que l'élément de contention soit photodurcissable par insolation au travers de l'applicateur.

Les moyens de l'état de l'art pour appliquer sur les surfaces dentaires une attelle dentaire ou élément de contention des dents, c'est à dire un élément allongé constituée de fibres, de particules, et de résine en l'état d'avant polymérisation sont difficiles et longs à mettre en oeuvre. L'application avec une spatule ou une bandelette maintenue par des moyens mécaniques, spatules, crochets, gouttières, fils, est difficile dans un contexte où les surfaces dentaires doivent rester exemptes d'humidité. Il en résulte des vides et un manque de cooptation de l'attelle sur les surfaces dentaires pouvant compromettre la pérennité du système.

De plus, lors de l'insolation pas à pas, progressant de dent à dent, la lumière initie la polymérisation d'une longueur d'attelle plus importante que la partie en contact avec la surface dentaire visée, et l'application de l'attelle partiellement durcie devient difficile sur la dent suivante.

### Objet de l'invention

L'invention a pour but d'éviter les inconvénients des attelles dentaires de l'état de l'art, en proposant une attelle dentaire facile à mettre en oeuvre et prête au collage.

Un premier objet de l'invention consiste à réaliser un élément de contention dentaire, constituée de fibres, de résine et de particules en l'état d'avant polymérisation et photopolymérisable, lequel élément de contention est muni de moyens d'adhésion immédiate et temporaire aux surfaces dentaires, pour autoriser une adhésion étroite et instantanée à toute surface même non plane. Ainsi, les moyens d'adhésion temporaire permettent à l'élément de contention dentaire selon l'invention de rester appliquée intimement aux surfaces dentaires par simple application sous légère pression pendant le temps de polymérisation de l'attelle et de la colle dentaire.

Un deuxième objet de l'invention consiste à fournir un élément de contention dentaire muni de moyens d'adhésion immédiate et temporaire aux surfaces dentaires, et étant complètement ou partiellement transparente aux rayons lumineux, pour permettre sa polymérisation et celle de la colle en une seule passe, sans l'utilisation des moyens mécaniques de l'état de l'art.

Un troisième objet de l'invention consiste à isoler l'élément de contention des fluides environnants grâce aux moyens d'adhésion immédiate et temporaire.

Un quatrième objet de l'invention consiste à réaliser un élément de contention dentaire portant des moyens d'adhésion immédiate et temporaire, complètement ou partiellement transparente aux rayons lumineux, et isolant l'élément de contention des fluides environnants, les moyens d'adhésion pouvant être retirés après photopolymérisation de l'élément de contention par simple traction. Lorsque l'on applique une traction sur les moyens d'adhésion, ils se séparent par pelage de l'élément de contention photopolymérisé et collé aux surfaces dentaires.

Selon l'invention, on utilise un applicateur déformable sans mémoire de forme, en matériau collant translucide capable d'adhérer temporairement aux surfaces dentaires sans préparation. L'applicateur peut être revêtu sur une ou plusieurs faces d'un matériau de protection non collant permettant sa préhension. Un élément allongé de contention est logé dans la structure collante de l'applicateur, et est constitué de fibres, de résine et de particules en l'état d'avant polymérisation et photopolymérisable. L'élément allongé peut être isolé de la structure collante de l'applicateur par un autre matériau isolant.

Selon un mode de réalisation préférentiel, l'élément de contention est associé à un applicateur souple et déformable, lequel est réalisé en un matériau à la fois tackifiant apte à adhérer temporairement aux surfaces dentaires, et conducteur de la lumière, tel que l'élément de contention soit photodurcissable au travers de l'applicateur par insolation. L'applicateur est doté d'une couche de protection non adhésive et translucide destinée à assurer sa préhension. L'élément de contention est logé dans une rainure de l'applicateur, la couche de protection étant située sur une face à l'opposé de la rainure. L'applicateur en matériau translucide tackifiant est bio compatible et ne laisse pas de résidus après son arrachement. La couche de protection non adhésive peut être armée de fibres de renforcement, ou être formée par un matériau non tissé. La couche de protection peut aussi être opaque, et facilement détachable au cours de l'insolation pour une polymérisation progressive, dent par dent.

La structure de l'applicateur déformable en matériau collant laisse passer les rayonnements lumineux destinés à amorcer la polymérisation de l'élément de contention inclus dans la structure collante tackifiant de l'applicateur. On utilise à titre d'exemple une lumière visible bleue, ayant une longueur d'onde comprise entre 435 et 490nm. L'applicateur peut être en un matériau tranparent, ou être en un matériau conducteur de lumière.

La résine tackifiante selon l'invention est choisie par exemple parmi le groupe constitué par les résines hydrocarbures solides ou liquides telles que les résines aliphatiques ou aromatiques, non hydrogénées ou hydrogénées totalement ou partiellement, par exemple ESCOREZ® 5300 d'EXXON et IMARVⓇ S 100 S d'IDEMITZU, les résines à base d'esters de colophane naturelle ou modifiée, par exemple polymérisée, notamment les esters de pentaérythritol ou de glycérol, par exemple UNITACⓇ R 100 L d'UNION-CAMP, les résines polyterpéniques ou polyterpéniques modifiées, par exemple WINGTACKⓇ de GOODYEAR et les résines α-méthyl-styrène, par exemple URATACKⓇ 68520 de DSM.

Des résines tackifiantes qui conviennent, sont par exemple la colophane, les esters de colophane, la colophane hydrogénée, les polyterpènes et dérivés, les résines de pétrole aromatiques ou aliphatiques, les résines cycliques hydrogénées, les dicyclopentadiènes. A titre de dérivés de la colophane on peut citer ceux obtenus par hydrogénation, deshydrogénation, polymérisation, estérification. Ces dérivés peuvent être utilisés tels quels ou sous forme d'esters de polyols, tels que les esters de pentaerythritol, polyéthylène glycol et glycérol.

Des plastifiants peuvent être utilisés dans les adhésifs de l'invention, par exemple des huiles minérales paraffiniques aromatiques ou naphténiques. Tous les plastifiants peuvent être utilisés pour essentiellement abaisser la viscosité et apporter du tack. La quantité de plastifiant peut être comprise entre 10 et 30 parties pour 100 parties du mélange, mais d'autres mélanges sont possibles.

La cire selon l'invention est généralement choisie parmi les cires d'origine pétrolière ou de synthèse, mais il peut s'agir de cires naturelles.

### Description sommaire des dessins

D'autres avantages et caractéristiques ressortiront plus clairement de la description qui va suivre de modes particuliers de réalisation de l'invention donnés à titre d'exemples non limitatifs et représentés aux dessins annexés, dans lesquels :
- la figure 1 représente une vue schématique de dessous de l'élément de contention dentaire selon l'invention ;
- la figure 2 est une vue en coupe selon la ligne 2-2 de la figure 1 ;
- la figure 3 montre une vue identiques de la figure 2 d'une variante de réalisation ;
- la figure 4 illustre la phase de positionnement de l'élément de contention sur les surfaces dentaires à renforcer ;
- la figure 5 montre la phase d'insolation pour la polymérisation de l'élément de contention ;
- la figure 6 représente la phase finale d'enlèvement de l'applicateur.

### Description d'un mode préférentiel de l'invention

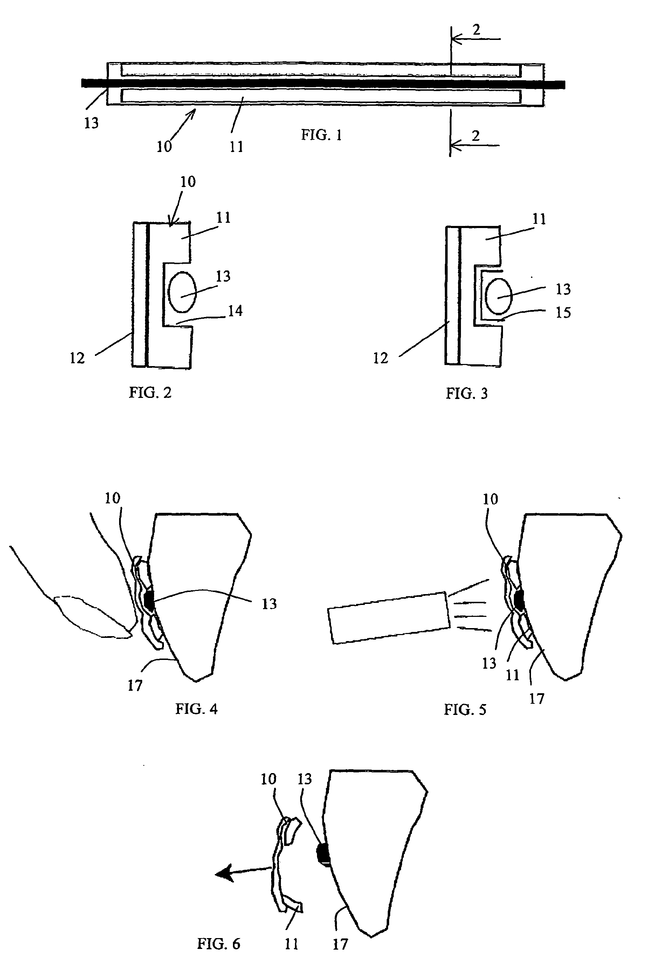

Sur les figures 1 et 2, un applicateur 10 est constitué d'une structure 11 déformable en matériau collant, présentant une section en U, et une face externe revêtue d'une couche de protection 12 en matériau non collant. Un élément de contention 13 allongé est logé dans une rainure 14 de la structure 11 collante.

L'élément de contention 13 est composé de fibres, de résine et de particules en l'état d'avant polymérisation et photopolymérisable.

Les fibres sont organisées sous diverses formes, faisceaux de fibres, fibres parallèles, éventuellement guipées, tissages, tricots, tresses, rubans ou n'importe quelle autre forme. La résine est une résine choisie en fonction de sa compatibilité avec les fibres utilisées. Une face de la structure collante de l'applicateur 10 est libre pour laisser affleurer l'élément de contention 13 photopolymérisable.

La structure de l'applicateur 10 déformable laisse passer les rayonnements lumineux destinés à amorcer la polymérisation de l'élément de contention 13 inséré dans la rainure 14 semi-ouverte de la structure 11 collante de l'applicateur. La face ouverte de la rainure 14 est dépourvue de résine.

La couche de protection 12 est de préférence en matériau translucide pour laisser passer le rayonnement lumineux.

Selon une variante, la couche de protection 12 peut aussi être opaque ou non translucide. Au cours de l'insolation, on enlève ladite couche dent par dent, de manière à obtenir une polymérisation progressive

On utilise à titre d'exemple une lumière visible bleue, ayant une longueur d'onde comprise entre 435 et 490nm. L'applicateur peut être en un matériau transparent, ou être en un matériau conducteur de lumière.

La variante de la figure 3 est similaire à celle de la figure 2, mais l'élément de contention 13 allongé est protégé de la structure 11 collante ou adhésive par un revêtement interne 15 translucide en matériau isolant recouvrant toutes les faces de la rainure 14.

Le dispositif de contention est appliqué sur la surface dentaire par déformation de l'applicateur 10, de manière à rester collé temporairement à la surface dentaire. L'élément de contention photopolymérisable logé dans l'applicateur 10 est plaqué sur la surface dentaire, et est ensuite polymérisé par insolation au travers de l'applicateur.

Sur la figure 4, l'élément de contention 13 est plaqué sur une surface dentaire 17 en exerçant une simple pression digitale sur l'applicateur 10 en matériau collant translucide.

Lors de la déformation de l'applicateur 10, il se maintient dans la nouvelle forme. Il n'a pas de mémoire de forme. Le matériau collant de la structure 11 souple de l'applicateur 10 est constitué en un matériau permettant d'adhérer immédiatement et de façon réversible aux surfaces dentaires. Le produit possède à cet effet au moins un agent tackifiant lui conférant une adhérence immédiate, mais non définitive à un support. L'effet tackifiant est obtenu par l'utilisation de matériaux tels que des résines de colophane, de leurs esters, de résines d'hydrocarbures, qui peuvent être estérifiées ou encore modifiées, de dispersions aqueuses de résines avec des parties de résines entre 50 et 70 % en poids, de toutes résines tackifiantes en général.

Il peut s'agir aussi de gomme, de gomme arabique, de cire collante, vinyle collant, et plus généralement de tout adhésif pourvu qu'il soit bio compatible et ne laisse pas de résidus après son arrachement.

Sur la figure 5, l'élément de contention 13 allongé adhérant à la surface dentaire 17, est insolé et polymérisé à travers la structure 11 collante translucide.

La figure 6 montre en fin de polymérisation, la phase d'enlèvement de la structure 11 collante de l'applicateur 10 laissant en place l'élément de contention 13 collé sur les surfaces dentaires 17.

## Revendications

1. Ensemble de contention dentaire comprenant un élément de contention (13) allongé en matériau composite à base de fibres et de particules préimprégnées avec une résine photopolymérisable, et destiné à former après polymérisation une attelle de renforcement de plusieurs dents entre elles, l'ensemble de contention dentaire comportant un applicateur (10) souple et déformable, lequel est réalisé en un matériau conducteur de la lumière, tel que l'élément de contention (13) soit photodurcissable par insolation au travers de l'applicateur (10), **caractérisé en ce que** le matériau de l'applicateur est tacktifiant apte à adhérer temporairement aux surfaces dentaires.

2. Ensemble de contention selon la revendication 1, **caractérisé en ce que** l'applicateur (10) est doté d'une couche de protection (12) non adhésive destinée à assurer sa préhension.

3. Ensemble de contention selon la revendication 2, **caractérisé en ce que** la couche de protection (12) est translucide.

4. Ensemble de contention selon la revendication 2, **caractérisé en ce que** la couche de protection (12) est en matériau opaque, apte à être détaché progressivement de l'applicateur (10) lors de l'insolation.

5. Ensemble de contention selon la revendication 2, **caractérisé en ce que** l'élément de contention (13) est logé dans une rainure (14) de l'applicateur (10), la couche de protection (12) étant située à l'opposé de la rainure (14).

6. Ensemble de contention selon la revendication 1, **caractérisé en ce que** l'applicateur (10) en matériau translucide tackifiant est bio compatible et ne laisse pas de résidus après son arrachement.

7. Ensemble de contention selon la revendication 6, **caractérisé en ce que** l'applicateur (10) est constitué en partie de colophane, de résine de colophane, de gomme, de cire collante, ou de vinyle collant.

8. Ensemble de contention selon la revendication 2, **caractérisé en ce que** la couche de protection (12) non adhésive est armée de fibres de renforcement.

9. Ensemble de contention selon la revendication 2, **caractérisé en ce que** la couche de protection (12) non adhésive est formée par une matière non tissé.

10. Ensemble de contention selon la revendication 1, **caractérisé en ce que** l'applicateur (10) est translucide et déformable sans mémoire de forme.

11. Ensemble de contention selon la revendication 1, **caractérisé en ce que** l'élément de contention (13) dentaire est isolé de l'applicateur (10) par un revêtement interne (15) isolant.

12. Ensemble de contention selon la revendication 1, **caractérisé en ce que** le matériau constitutif de l'applicateur (10) est translucide ou conducteur de lumière, et est adapté pour être traversé par une lumière visible bleue, ayant une longueur d'onde comprise entre 435 et 490nm.

## Claims

1. Dental setting device comprising an elongated setting element (13) composed of composite material based on fibres and particles which are preimpregnated with a photopolymerizable resin and intended to form, after having been polymerized, a reinforcement splint for several teeth, the dental setting device comprising a flexible and malleable applicator (10) which is made from a light-guiding material, such that the setting element (13) is photo-hardenable by exposure through the applicator, **characterized in that** the material of the applicator is tackifying and able to temporarily adhere to the dental surfaces.

2. Setting device according to claim 1, **characterized in that** the applicator (10) is provided with a non-adhesive protective layer (12) designated for ensuring its grasp.

3. Setting device according to claim 2, **characterized in that** the protective layer (12) is translucid.

4. Setting device according to claim 2, **characterized in that** the protective layer (12) is made of an opaque material able to be progressively detached from the applicator (10) while being exposed.

5. Setting device according to claim 2, **characterized in that** the setting device (13) is housed in a groove (14) of the applicator (10), the protective layer (12) being provided opposite to the groove (14).

6. Setting device according to claim 1, **characterized in that** the applicator (10) made from tackifying translucid material is biocompatible, leaving no residuals after its removal.

7. Setting device according to claim 6, **characterized in that** the applicator (10) consists partially from colophony, colophony resin, gum, sticky wax or sticky vinyle.

8. Setting device according to claim 2, **characterized in that** the non-adhesive protective layer (12) is reinforced with reinforcement fibres.

9. Setting device according to claim 2, **characterized in that** the non-adhesive protective layer (12) is composed by a non-woven fabric.

10. Setting device according to claim 1, **characterized in that** the applicator (10) is translucid and malleable without shape memory.

11. Setting device according to claim 1, **characterized in that** the dental setting element (13) is separated from the applicator (10) by means of an insulating inner coating (15).

12. Setting device according to claim 1, **characterized in that** the material the applicator (10) is made from is translucid or light-guiding and apt for being penetrated by a blue visible light of a wavelength of 435 to 490 nm.

## Patentansprüche

1. Zahnhaltevorrichtung, die ein längliches Halteelement (13) aus Verbundmaterial auf Faserbasis und mit einem fotopolymerisierbaren Harz vorimprägnierten Partikeln umfasst und dazu bestimmt ist, nach der Polymerisation eine Verstärkungsschiene für mehrere Zähne untereinander zu bilden, wobei die Zahnhaltevorrichtung einen weichelastischen und verformbaren Applikator (10) umfasst, der aus lichtleitendem Material besteht, sodass das Zahnhalteelement (13) strahlungshärtbar durch Belichtung durch den Applikator hindurch ist, **dadurch gekennzeichnet, dass** das Material des Applikators klebend und geeignet ist, vorübergehend an den Zahnoberflächen zu haften.

2. Haltevorrichtung nach Anspruch 1, **dadurch gekennzeichnet, dass** der Applikator (10) mit einer nicht haftenden Schutzschicht (12) versehen ist, die sein Greifen ermöglichen soll.

3. Haltevorrichtung nach Anspruch 2, **dadurch gekennzeichnet, dass** die Schutzschicht (12) lichtdurchlässig ist.

4. Haltevorrichtung nach Anspruch 2, **dadurch gekennzeichnet, dass** die Schutzschicht (12) aus lichtdichtem Material besteht, das geeignet ist, bei der Belichtung schrittweise vom Applikator (10) gelöst zu werden.

5. Haltevorrichtung nach Anspruch 2, **dadurch gekennzeichnet, dass** das Halteelement (13) in einer Nut (14) des Applikators (10) angeordnet ist, wobei sich die Schutzschicht (12) entgegengesetzt zur Nut (14) befindet.

6. Haltevorrichtung nach Anspruch 1, **dadurch gekennzeichnet, dass** der Applikator (10) aus lichtdurchlässigem, klebendem Material biokompatibel ist und nach seiner Entfernung keinerlei Rückstände hinterlässt.

7. Haltevorrichtung nach Anspruch 6, **dadurch gekennzeichnet, dass** der Applikator (10) zum Teil aus Kolophonium, Kolophoniumharz, Gummi, klebendem Wachs oder klebendem Vinyl besteht.

8. Haltevorrichtung nach Anspruch 2, **dadurch gekennzeichnet, dass** die nicht haftende Schutzschicht (12) durch Verstärkungsfasern verstärkt ist.

9. Haltevorrichtung nach Anspruch 2, **dadurch gekennzeichnet, dass** die nicht haftende Schutzschicht (12) von einem Faservlies gebildet wird.

10. Haltevorrichtung nach Anspruch 1, **dadurch gekennzeichnet, dass** der Applikator (10) lichtdurchlässig und verformbar ohne Formgedächtnis ist.

11. Haltevorrichtung nach Anspruch 1, **dadurch gekennzeichnet, dass** das Zahnhalteelement (13) vom Applikator (10) durch eine isolierende Innenbeschichtung (15) getrennt ist.

12. Haltevorrichtung nach Anspruch 1, **dadurch gekennzeichnet, dass** das den Applikator (10) bildende Material lichtdurchlässig oder lichtleitend und geeignet ist, von einem sichtbaren blauen Licht durchstrahlt zu werden, das eine Wellenlänge von 435 bis 490 nm hat.
